# EUROPEAN PATENT SPECIFICATION

(11) **EP 0 191 066 B1**
(45) Date of publication and mention of the grant of the patent: **24.02.1993**
(21) Application number: 85904075.0
(22) Date of filing: 14.08.1985
(51) Int. Cl.: A01K 67/00, A01N 63/00

(54) **LIQUID CULTURE OF NEMATODES**
FLÜSSIGES ZÜCHTEN VON NEMATODEN
CULTURE LIQUIDE DE NEMATODES

(30) Priority: 14.08.1984 AU 6566/84
(43) Date of publication of application: 20.08.1986
(73) Proprietor: ECOGEN-BIO INC., Langhorne, Pennsylvania 19047 (US)
(72) Inventor: PACE, Gary, William, Pymble, NSW 2073 (AU); GROTE, Wilfred, Maroubra, NSW 2035 (AU); PITT, David, Edward, North Turramurra, NSW 2074 (AU); PITT, Janice, Margaret, North Turramurra, NSW 2074 (AU)
(74) Representative: Moore, Derek
(86) International application number: AU8500199
(87) International publication number: WO8601074

(56) References cited:
- AU-B- 1 662 176
- AU-B- 5 648 880
- DE-A- 1 900 916
- SU-A- 803 927
- US-A- 4 334 498
- US-A- 4 417 545
- JOURNAL OF ECONOMIC ENTOMOLOGY, vol. 75, no. 4, August 1982, pages 701-703, College Park, Maryland, US; C.S. CREIGHTON et al.: "Mass rearing a mermithid nematode, Filipjevimermis leipsandra (mermithida: mermithidae) on the banded cucumber beetle (coleoptera: chrysomelidae)
- J. M. Webster et al, 10th International Congress of Plant Protection 1983, Volume 2 "Enhancing the Use of Entomogenous Nematodes in Controlling Insect Pests" published Croydon U.K. British Crop Protection Council 1983
- STOLL, Annals New York Academy of Sciences, 1959, pp. 126-136
- J. R. VANFLETEREN, Experientia 32, 1976, p. 1087-1088
- R. A. Bedding, Nematologica V. 27 (1) Jan 1981 "Low Cost in vitro Mass Production of Neoaplectana and Heterorhabditis species (Nematoda) for field control of Insect Pests" pp. 109-114

## Description

### Introduction

The present invention relates to the large scale production of entomopathogenic nematodes by a liquid culture method.

### Background Art

Entomopathogenic nematodes are known to be useful in the biological control of a wide range of insect pests. However the commercial use of nematode bioinsecticides has been limited by the inability to produce the nematodes on a commercial scale and at a cost comparable with chemical insecticides. The large scale production of nematodes is therefore of great commercial significance.

Entomopathogenic nematodes go through various developmental phases in their life cycle. In the natural environment of the host insect haemocoel, the infective stage (J3) nematodes exsheath, expell their bacteria, increase in metabolic activity and commence feeding. Bacteria of the nematode-specific strain of Xenorhabdus nematophilus or X. luminescens are excreted by the nematodes and multiply in the haemocoel. The nematodes grow to adult size and reproduce, giving eggs, which develop through J1, J2 stages to produce the next generation of infective stage (J3) nematodes, after one to three weeks. Infective nematodes leave the insect cadaver and seek a new host. Bacterial septicema is the primary cause of larval death. However, growth and reproduction of the nematodes, or the production of insecticidal substances by the nematodes can cause the death of the insect larvae, in the absence of the symbiotic bacteria.

A major limitation to the commercial exploitation of entomopathogenic nematodes for control of economically important insect pests has been the inability to produce large numbers of the infective (dauer stage) nematodes by in vitro culture. In vivo culture using Galleria larvae in particular has provided researchers with sufficient material only for laboratory and limited field trial experiments.

U.S. Patent 4,334,498 discloses a method for in vitro mass rearing using homogenized offal on a sponge matrix. This method and the overall approach it represents, currently indicate state-of-the-art for nematode cultivation and of the various methods, is the most amenable to large scale operation. The need for solid support phases in a growth medium is most favoured by nematologists. Bedding (Ann. appl. Biol. (1984), 104, 117-120) teaches the need for a solid matrix to promote aeration of the environment in nematode culture. He points out that even gentle agitation almost totally suppresses nematode development, which supports the belief, commonly shared in the art, that liquid culture on a large scale would not be viable. However, it would be very desirable to be able to culture nematodes on a large scale in liquid culture. Liquid culture is far easier to scale up to produce large quantities of nematodes. Liquid culture also facilitates the maintenance of aseptic conditions and the monitoring and harvesting of the nematodes.

Although the growth of entomopathogenic nematodes has been established in liquid culture, this has only been achieved in small volumes of less than about 1 litre. For example in the course of nutritional studies liquid culture volumes of less than 100 ml were used by Stoll 1953 and 1961, Jackson 1962, 1965a and 1965b and 1973, Hansen et al. 1967, Lower and Buecher 1970, and Buecher et al. 1970. Hansen et al. 1967 and Tarakanow 1983 used thin liquid film nematode culture.

In a lecture given in 1959, Stoll dicussed developments in this area, particularly his research, and disclosed the details of small scale (10ml) liquid culture growth, 1959 Annals of the New York Academy of Science pages 126-136.

No research has been published to date on large scale (i.e. over 1 litre) submerged liquid culture studies with entomopathogenic nematodes using conventional fermenter systems available.

The liquid cultures referred to above yielding low concentrations of juvenile infective nematodes (several thousand per mL), and consisted of mixtures of infective and non-infective juvenile and adult entomopathogenic nematodes. Only the infective stage nematodes are effective in the control of insects and survive well in storage and application. The presence of a mixture of life stages also appears to decrease the life of infective stage nematodes. Thus a process producing a mixture of life stages is undesirable economically.

The small scale nematode liquid culture described in the literature, did not encounter the problems characteristic of large scale culture, for example, providing enough oxygen to the mixed culture of nematodes and bacteria, and removing CO₂ and other volatile products of metabolism.

Our work has shown that culture in conventional, (flat blade) impeller equipped fermenters, increased nematode yields, when compared to published yields from shake flask culture, but was still uneconomical due to high shear stress encountered in this conventional fermenter.

Further work by the inventors with a low shear (internal draft tube) air lift fermenter revealed that the lift provided by bulk flow velocity at the base of the draft tube, is not sufficient to suspend heavy female nematodes.

The present inventors have found that large scale liquid culture of entomopathogenic nematodes, to produce high concentrations of relatively pure infective stage nematodes, is possible so long as certain critical parameters are followed for the preparation of the culture medium and the culturing process itself.

### Disclosure of the Invention

The present invention consists of a method of producing, on a large scale, a high concentration of substantially pure infective stage nematodes, comprising: providing, on a large scale, a liquid culture medium containing entomopathogenic nematodes in a large scale reactor; culturing said medium to produce, on a large scale, a high concentration of substantially pure infective stage nematodes, by agitating and aerating said medium, characterised by agitating and aerating said medium under low shear conditions by blowing air downwards towards the bottom of said reactor thereby sweeping up heavy adult nematodes from said bottom and suspending said heavy nematodes in said medium.

Further, the liquid cultures can be grown where low shear forces are obtained by culturing the nematodes in a low shear reactor.

In one form the invention encompasses a method for the large scale liquid culture of entomopathogenic nematodes for producing high concentrations of relatively pure infective stage nematodes, whereby the culture medium for said nematodes is pre-inoculated with the nematode's symbiotic bacteria, which have been grown or processed in such a manner so as to reduce compounds which are inhibitory or toxic to said nematodes.

The nematodes symbiotic bacteria exist in two distinct forms referred to as primary and secondary. Preferably the bacteria will be taken from a strain which stably maintains primary characteristics after repeated subculture. The primary form of the symbiotic bacteria can revert to the secondary form. Presence of the secondary form of the bacterium during nematode growth and reproduction leads to reduced nematode yields in solid culture (Bedding 1984). We have observed yield reductions in liquid culture when secondary Xenorhabdus were present. Previous attempts at liquid culture did not use the symbiotic bacteria.

In one form, the nematode culture contains at the time of nematode addition, from 5 to 10 g L⁻¹ (dry weight) of bacterial cells per litre of medium.

In another form of the present invention the nematodes are grown in axenic culture and the primary form bacteria are added when the new generation of nematodes are in the J2 stage.

It will be clear from the above that nematodes may be cultured either axenically or in the presence of symbiotic bacteria. Addition of the symbiotic bacteria is important to maximise the infectivity of nematodes against insects.

A critical parameter to this process of culturing said nematodes are the shear forces to which the nematodes are subjected to whilst in the reactor.

Most sensitive to shear is the reproductive process of the nematodes. This process requires that the male coils himself around the larger female. Large egg bearing and laying females, and recently hatched J1 stage nematodes are also easily damaged by shear. Our work has established that shear (from a flat blade impeller) expressed as impeller tip velocity (ND) of 1 ma ⁻¹ or greater leads to the disruption of adult females. Preferably shear should be less than 0.3 ms⁻¹ for maximum yield.

The aeration and agitation systems of a conventional stirred tank reactor were removed from the reactor and a purpose designed aeration system installed. This aeration sparger blows air downwards against the bottom of the vessel to sweep up and lift heavy adult nematodes. This sparger operates in the reverse direction when compared to aeration systems used in conventional bubble column fermenters, which blow upwards.

Advantages of the liquid culture method in conventional fermentation vessels over state-of-the-art solid culture are firstly, that aseptic samples can be withdrawn and analysed, for example to test for contamination, or to determine when the culture should be treated to enhance formation of infective nematodes. Secondly, monitoring of important parameters such as temperature, pH, oxygen and carbon dioxide levels is possible, and can be used to predict the state of the culture. Thirdly, aseptic additions can be made to the culture, for example the addition of Xenorhabdus bacteria at a critical stage in axenic cultures. Fourthly, because liquid culture can be closely monitored, the inoculation stages required for a large volume fermentation can be tested for contamination, and most important, can be harvested and used as inoculum after approx. one week culture time, before the nematodes are in the infective stage, leading to a saying of six weeks for a fermentation requiring three inoculum stages. Non infective nematodes have not been used as inoculum in nematode production before.

Further important, whilst not essential, parameters concern the culture medium. The medium should preferably contain homogenized material of animal origin such as chicken guts or kidney of ox or pig. The medium composition should be adjusted to assist in stimulating the growth of the particular species of nematodes and its bacterial symbiont, to be cultured, and the progression of the nematode into the infective form.

It is also important to maintain aseptic conditions throughout the preparation of the bacterial inoculum and nematode growth to prevent undesirable microbial growth.

In a further form of the present invention, there is provided a process by which large scale liquid culture of nematodes, particularly entomopathogenic nematodes, results in a significant proportion of mature dauer stage infective nematodes, characterised in that the culture medium is substantially diluted or replaced with non-nutritive aqueous medium or water, while aeration and agitation are maintained, at the stage when third stage juveniles are present.

Preferably the temperature is also lowered to 15°C or below.

Notwithstanding other forms which may fall within the broad form of the present invention, the following examples illustrate the growth of different nematode strains in large scale liquid culture. It should be noted that the invention is applicable to all strains of entomopathogenic nematodes, although the examples illustrate only selected strains.
Preferred embodiments of the invention will now be described by was of example.

### Example 1:

Growth of Steinernema feltiae (All) (S.f.A) in a 20 litre Braun E series fermenter operating as a gas lift fermenter in the form of a bubble column.

A bacterial inoculum of S.f.M symbiont Xenorhabdus nematophilus (N.f.M./1) was prepared, by transferring one colony of the primary form of the bacterium, from an indicator plate, (nutrient agar with bromothymol blue, 25 mg L⁻¹ and 2,3,5-triphenyltetrazolium chloride, 40 mg L⁻¹, Akhurst 1980) into 200 mL glycerol-yeast extract-salts (GYS) medium. After 24 hours (H), at 28°C, shaker at 120 rpm, the culture was transferred aseptically to the 20 litre fermenter.

The fermenter already contained 8 litres of sterile medium which was made up of 100g L⁻¹ ox kidney homogenate and 10gL⁻¹ yeast extract in deionised water, the sterile medium was sterilised by passing it through a continuous flash sterilizer 1 minute at 155°C. The potential cost reduction of flash sterilization can be used advantageously with large scale liquid culture systems, alternatively, the sterile medium can be sterilised in situ.

Defined media, semidefined media and media based on different types of offal also support nematode growth and reproduction at varying yields.

The fermenter parameters were set as follows:
Temperature 28°C, pH 6.8 (maintained with 20% w/v H₃PO₄, and 20% w/v NaOH), aeration and agitation to maintain 50% oxygen saturation, using automatic set point control.

A standard 20 litre Braun fermenter vessel was used however, impeller shaft, impellers and gas sparger were removed. In their place a specifically designed sparger was installed, to provide both aeration and agitation i.e. lift and suspend heavy adult nematodes.

The sparger consisted of three interconnected rings made from 6mm tubing, and was installed 20mm above the floor of the vessel. The outermost ring, diameter 150mm, had 2mm holes spaced 10mm apart. The holes aimed at an angle of 45° from the vertical downward at the sides of the vessel. The two inner rings of 100mm and 50mm diameter had also 2mm holes, 10mm apart, but aimed directly downwards. This design is applicable to flat bottomed vessels and prevents nematode settling effectively in a minimum shear environment. The air flow rate was set to a minimum flow rate of 10 NL min⁻¹, but allowed to increase if the dissolved oxygen level fell below 20% of saturation.

After 24H fermenter time a monoculture of S.f.M harvested using aseptic technique, from a solid culture flask, and in the infective stage, was added aseptically to the fermenter to give a concentration of 2000 juvenile (J3) nematodes mL⁻¹.

Fermeter parameters were reset to 23°C, no pH control (the pH increases from 6.8 to 8.7), stirring rate 180 rpm (which was close to the minimum of 150 rpm obtainable with the fermeter) and aeration rate variable to maintain at least 20% oxygen saturation. Foam was not controlled, instead an expansion vessel was installed in the gas outlet line. Samples were withdrawn (aseptically) from the shaker flask and the fermenter, to enumerate primary and secondary Xenorhabdus and later the nematodes by viable and direct microscope counts.

In the fermenter the nematodes developed from the juvenile infective (dauer) stage and reached the adult (J4) stage after about 80H. Copulation was observed at about this time followed by further development and the observation of egg bearing females at c.a. 100H.

Between 100 and 180H free eggs and J1 stage juvenile nematodes were observed. The culture reached 40,000 mL⁻¹ J3 stage juvenile nematodes after 240H with 90% viability.

After a further 10 days in the fermenter, (to increase the number of infective nematodes) the nematodes were harvested by settling and washing.

The nematode bioinsecticide was bioassayed by adding 5000 infective nematodes to 20 Lucilia cuprina 3rd instar larvae, the control were solid culture reared nematodes. The liquid culture nematodes performed as well as the control, by killing on average 15 out of 20 presented larvae.

Nematode yield was 90,000 mL⁻¹ again with a high level of infectivity.

### Example 2:

Use of inoculum stages using nematodes not in the infective stage.

A large scale nematode liquid culture would require an inoculum build-up over several stages of liquid culture. Only infective stage (J3) juveniles have been used as the inoculum in the state-of-the-art method.

If liquid nematode culture takes three weeks, then nine weeks would be required to provide the three inoculum building up stages required for a large volume liquid culture.

Inoculum build up was simulated in shaker flask culture, 150mL culture in 500mL conical flasks, shaking at 110rpm at 22°C. Strain S.f.M, media and conditions as outlined in Example 1 were used.

The nematodes were subcultured sequentially five times, after about one week culture time, when J1 and J2 stage juveniles were present.

These cultures yielded between 60,000 and 90,000 juveniles mL⁻¹ and when left to reach the infective stage, were found to be equivalent in infectivity to the control.

Time for inoculum building up can thus be reduced from nine to three weeks.

### Example 3:

Growth of different entomopathogenic nematodes in liquid culture.

Ten litre fermenter and 500mL shake flask cultures were carried out under conditions described above.

The kidney-yeast extract medium was sterilized by the standard 20min. at 121°C, 15 psi, batch method, by flash sterilization, or by microwaving for 10min, (100mL⁻¹ of medium) in a microwave oven 650W, freq. 2450MHz. Flash sterilization and microwaving can be operated in the continuous mode leading to cost reduction for large scale operation.

Liquid culture for different species or strains of entomopathogenic nematodes has not yet been optimized, thus still higher yields can be expected.

### Results:

| Nematode | Maximum Yield ML⁻¹ | |
|---|---|---|
| | Shake Flask | Fermenter |
| s.f. (Mexican) | 120,000 | 70,000 |
| s.f. (All) | 190,000 | 90,000 |
| s.f. (Agriotis) | 125,000 | 65,000 |
| s. bibionis | 70,000 | 63,000 |
| s. glaseri | 66,000 | 40,000 |
| Heterorhabditis heliothidis | 20,000 | 15,000 |

Infective (dauer stage) juveniles are harvested at levels of 60 to 90% from these strains be replacing the rich medium with distilled water lowering the temperature to 15^{o}C and sparging with air, at a critical stage in the culture, which occurs when the first nonfeeding doubly ensheathed, resistant, infective (dauer) nematodes appear.

The above examples serve to illustrate that large scale submerged liquid culture is feasible for nematode production. Although only demonstrated in 20L vessels, it is recognised in the art that results achieved at this scale can be scaled up to commercial volumes, e.g., greater than three cubic metres. Optimization of the physical environment of the nematodes is central in maximising development, copulation, fecundity and formation of infectives.

Entomopathogenic nematodes produced by the method of the invention may be used as broad spectrum biological insecticides.

The nematodes may be administered by known methods such as spraying on crops, injecting into tree trunk, spraying on soil and applying to soil in solid form such as pellets, in diluted or undiluted form.

### References:

Ackerman, E., (1962) Biophysical Science, Prentice Hall, Engelwood Cliffs, N.J.
Akhurst, R.J. (1980) J. Gen. Microbial. 121:303-309
Bedding, R.A., (1984) Ann. appl. Biol. 104:117-120
Buecher, E.J., Hansen, E.K., and Yarwood, E.A., (1970) Nematologia 16:403-409.
Hansen, E., Yarwood, E.A., and Jackson, G.J. (1967) 42nd Ann. Meeting Am. Soc. Parasitol.
Jackson, G.J. (1962) Exp. Parasitol. 12:25-32.
Jackson, G.J. (1965) Parasitol 55:571-578.
Jackson, G.J. and Siddiqui, W.A., (1965) J. of Parasitol. 51:727-730.
Jackson, G.J. (1973) Exp. Parasitol. 34:111-114.
Lower, W.M.R., and Buecher, E.J. (1970) Nematologia 16:563:566.
Middler, M., and Finn, R.K., (1966) Biotech. and Bioeng. 23:71-84.
Stoll N., 1953) J. Parasitol. 39:422-444.
Stoll, N., (1959) Ann. New York Academy of Science, vol 77 page 126-137.
Stoll, N., (1961) J. Helminthol. Lisper Suppl. 169-174.
Tarakanow, V.I., (1983) Biocontrol News and Information 4:69.

## Claims

1. A method of producing, on a large scale, a high concentration of substantially pure infective stage nematodes, comprising:
providing, on a large scale, a liquid culture medium containing entomopathogenic nematodes in a large scale reactor;
culturing said medium to produce, on a large scale, a high concentration of substantially pure infective stage nematodes, by
agitating and aerating said medium, characterised by agitating and aerating said medium under low shear conditions by blowing air downwards towards the bottom of said reactor thereby sweeping up heavy adult nematodes from said bottom and suspending said heavy nematodes in said medium.

2. A method according to claim 1, wherein the shear forces are less than 1.0 ms⁻¹.

3. A method according to claim 1, wherein the shear forces are less than 0.3 ms⁻¹.

4. A method according to any one of claims 1 to 4, wherein low shear forces are obtained by culturing the nematodes in a low shear reactor.

5. A method according to claim 4, wherein the low shear reactor is a bubble column fermenter.

6. A method according to claim 1, wherein the culture medium is preinoculated with the nematode's symbiotic bacterium, said bacterium having been grown or processed in such a manner that compounds produced by said bacterium, which compounds are inhibitory on toxic to said nematodes, are reduced.

7. A method according to claim 6, wherein the bacteria are taken from a strain which stably maintains primary characteristics after repeated subculture.

8. A method according to claim 1, wherein the nematodes are grown in axenic culture and the primary form of the nematodes' symbiotic bacterium is added when the nematode have developed to at least the J2 stage.

9. A method according to claim 1, wherein subculturing is performed after approximately 1 week culture time when J1 and J2 stage juveniles predominate.

## Patentansprüche

1. Verfahren zur Herstellung in großem Maßstab einer hohen Konzentration von Nematoden eines im wesentlichen reinen infektiösen Stadiums durch:
Bereitstellung in großem Maßstab eines flüssigen Kulturmediums, enthaltend entomopathogene Nematoden, in einem Reaktor großen Maßstabes;
sowie Kultivierung des genannten Mediums zur Herstellung in großem Maßstab einer hohen Konzentration von Nematoden eines im wesentlichen reinen infektiösen Stadiums durch Rühren und Belüften des genannten Mediums, dadurch gekennzeichnet, daß man das genannte Medium unter niedrigen Scherbedingungen rührt und belüftet, indem man Luft abwärts in Richtung auf den Boden des Reaktors bläst, wodurch man schwere adulte Nematoden von dem genannten Boden aufnimmt und die genannten schweren Nematoden in dem Medium suspendiert.

2. Verfahren nach Anspruch 1, bei dem die Scherkräfte weniger als 1,0 ms⁻¹ betragen.

3. Verfahren nach Anspruch 1, bei dem die Scherkräfte weniger als 0,3 ms⁻¹ betragen.

4. Verfahren gemäß einem beliebigen der Ansprüche 1 bis 3, bei dem niedrige Scherkräfte erhalten werden, indem man die Nematoden in einem Reaktor mit niedrigen Scherkräften kultiviert.

5. Verfahren nach Anspruch 4, bei dem der Reaktor mit niedrigen Scherkräften ein Blasensäulen-Fermenter ist.

6. Verfahren nach Anspruch 1, bei dem das Kulturmedium mit dem symbiotischen Bakterium der Nematoden präinokuliert wird, wobei das genannte Bakterium in einer Weise gezüchtet oder zubereitet worden ist, daß von dem Bakterium gebildete Verbindungen, die gegenüber den genannten Nematoden inhibitorisch oder toxisch sind, verringert werden.

7. Verfahren nach Anspruch 6, bei dem die Bakterien von einem Stamm genommen wurden, der primäre Charakteristiken nach wiederholter Subkultivierung in stabiler Weise beibehält.

8. Verfahren nach Anspruch 1, bei dem die Nematoden in axenischer Kultur gezüchtet werden und die primäre Form des symbiotischen Bakteriums der Nematoden zugesetzt wird, wenn die Nematoden sich mindestens bis zu dem J2-Stadium entwickelt haben.

9. Verfahren nach Anspruch 1, bei dem die Subkultivierung nach einer Züchtungszeit von annähernd einer Woche vorgenommen wird, wenn die Juvenilen der Stadien J1 und J2 vorherrschen.

## Revendications

1. Un procédé de production, sur une grande échelle, d'une concentration élevée de nématodes à un état infectieux sensiblement pur comprenant le fait de :
réaliser, sur une grande échelle, un milieu de culture liquide contenant des nématodes entomopathogènes dans un réacteur à grande échelle ;
mettre en culture ledit milieu pour produire, à grande échelle, une concentration élevée de nématodes à un état infectieux sensiblement pur, par
agitation et aération dudit milieu, caractérisé par le fait d'agiter et d'aérer ledit milieu sous des conditions de faible cisaillement en soufflant de l'air vers le bas en direction du fond dudit réacteur de manière ainsi à amener vers le haut par balayage des nématodes adultes lourds à partir dudit fond et mettre en suspension lesdits nématodes lourds dans ledit milieu.

2. Un procédé selon la revendication 1, dans lequel les forces de cisaillement sont inférieures à 1,0 ms⁻¹.

3. Un procédé selon la revendication 1, dans lequel les forces de cisaillement sont inférieures à 0,3 ms⁻¹.

4. Un procédé selon l'une quelconque des revendications 1 à 3, dans lequel les forces faibles de cisaillement sont obtenues en cultivant les nématodes dans un réacteur à faible cisaillement.

5. Un procédé selon la revendication 4, dans lequel le réacteur à faible cisaillement est un fermenteur à colonne de barbotage.

6. Un procédé selon la revendication 1, dans lequel le milieu de culture est inoculé au préalable avec le bactérium symbiotique des nématodes, ledit bactérium ayant été élevé et traité de telle manière que des composés produits par ledit bactérium, composés qui sont inhibiteurs ou toxiques vis-à-vis desdits nématodes, sont réduits.

7. Un procédé selon la revendication 6, dans lequel les bactéries sont prises depuis une souche qui conserve de manière stable des caractéristiques primaires après sous-culture répétée.

8. Un procédé selon la revendication 1, dans lequel les nématodes sont élevés dans une culture axénique et la forme primaire du bactérium symbiotique des nématodes est ajoutée lorsque les nématodes se sont développés jusqu'à au moins l'état J2.

9. Un procédé selon la revendication 1, dans lequel la sous-culture est effectuée après un temps de culture d'environ une semaine lorsque prédominent des juvéniles d'état J1 et J2.
